**Europäisches Patentamt**

**European Patent Office**

**Office européen des brevets**

(11) Veröffentlichungsnummer: **0 169 311**
**B1**

(12) **EUROPÄISCHE PATENTSCHRIFT**

(45) Veröffentlichungstag der Patentschrift:
**18.10.89**

(21) Anmeldenummer: **85104651.6**

(22) Anmeldetag: **17.04.85**

(51) Int. Cl.⁴: **A 61 B 17/22**

(54) Ortungs- und Positioniervorrichtung.

(30) Priorität: **21.07.84 DE 3427001**

(43) Veröffentlichungstag der Anmeldung:
**29.01.86 Patentblatt 86/5**

(45) Bekanntmachung des Hinweises auf die Patenterteilung:
**18.10.89 Patentblatt 89/42**

(84) Benannte Vertragsstaaten:
**BE FR GB IT NL SE**

(56) Entgegenhaltungen:
**AT-B- 357 661**
**DE-A- 2 643 126**
**DE-A- 3 122 056**
**DE-A- 3 220 751**
**DE-B- 2 351 247**
**DE-B- 2 722 252**
**US-A- 4 399 822**

**PROSPEKT: DORNIER NIERENLITHOTRIPTER, ZMV
207-84035000**

(73) Patentinhaber: **DORNIER GMBH, Postfach 1420,
D-7990 Friedrichshafen 1 (DE)**

(72) Erfinder: **Hepp, Wolfgang, Dr., Hardtstrasse 6,
D-7997 Immenstaad (DE)**
Erfinder: **Restle, Karl-Heinz, Herrgottsweiler 3,
D-7992 Tettnang (DE)**
Erfinder: **van Rijn, Dick, Gartenstrasse 9,
D-7991 Eriskirch (DE)**
Erfinder: **Wess, Othmar, Dr., Hersbergweg 6,
D-7997 Immenstaad (DE)**

(74) Vertreter: **Landsmann, Ralf, Dipl.-Ing., c/o DORNIER
GMBH Postfach 1420, D-7990 Friedrichshafen 1 (DE)**

## Beschreibung

Die Erfindung betrifft eine Ortungs- und Positioniervorrichtung für die berührungsfreie Zerkleinerung von Konkrementen in Körpern von Lebewesen nach dem Oberbegriff des Anspruchs 1.

Aus der DE-PS 2 351 247 ist eine Einrichtung zum Zerkleinern von im Körper eines Lebewesens befindlichen Konkrementen mit einer Fokussierungskammer, die Teil eines Rotationsellipsoids ist und in derem einen Brennpunkt Stosswellen durch Funkenentladung erzeugbar sind, bekannt. Damit ist eine Zerkleinerung von zum Beispiel Nierensteinen ohne operativen Eingriff oder Einführung von Sonden möglich.

In den Nierenlithotriptern, die Anfang 1984 von der Dornier System GmbH ausgeliefert wurden, wird der Nierenstein mittels zweier Röntgenabbildungssysteme in seiner Grösse und seiner Lage im Körper des Patienten lokalisiert (geortet) (Prospekt: Dornier Nierenlithotripter, ZMV 207–840335000). Der Patient wird gegenüber dem Stosswellensystem so verschoben (positioniert), dass der Stein im zweiten Brennpunkt der Fokussierungskammer liegt. Dann wird der Stein von fokussierten Stosswellen zu einem feinen Gries zerkleinert, der auf natürliche Weise aus dem Körper herausgeschwemmt wird. Dabei sind die Vorrichtungen zur Erzeugung des Stosswellenfeldes und die Vorrichtungen zur Ortung so angebracht, dass sie sich räumlich nicht behindern. Die zwei Röntgengeräte zum räumlichen Orten sind neben dem Reflektor angeordnet. Die beiden Zentralstrahlen der zwei Röntgenortungsgeräte schneiden die Reflektorachse unter je einem Winkel von ca. 40° nahe dem Steinort. Diese Lösung mit zwei Röntgengeräten ist relativ aufwendig, ausserdem sind im Reflektor Ausschnitte für den Durchtritt der Röntgenstrahlen notwendig. Die im Bereich der Röntgenstrahlung liegenden Wasserstrecken beeinflussen die Aufnahmequalität oder machen das Einbringen von aufblasbaren Bälgen erforderlich.

Als Alternative für die Ortung und Positionierung bietet sich Ultraschall aus verschiedenen Gründen an. In der praktischen Positionierung von Konkrementen ist jedoch hinsichtlich Zeitbedarf der Ultraschall dem Röntgen unterlegen, weil der Suchprozess mit einem am Stosswellenlithotripter fixierten Schwinger schwierig ist.

Neben anderen Ursachen ist in der Hauptsache die Beschränkung durch die Ultraschall-Eintrittsfenster des menschlichen Körpers von Bedeutung. Es ist nicht nur von grösster Wichtigkeit, ein Eintrittsfenster für den Ultraschall zu finden, sondern auch in der richtigen Richtung durch das Fenster zu schauen (Schlüssellocheffekt). Weiterhin stört die Bewegung der Organe aufgrund der Atemverschieblichkeit bzw. anderer Einflüsse.

Aufgabe der Erfindung ist es, eine Ortungs- und Positioniervorrichtung für die berührungsfreie Zerkleinerung von Konkrementen in Körpern von Lebewesen zu schaffen, die Echtzeitbeobachtung erlaubt, die kostengünstig ist, die eine schnelle und zuverlässige Ortung auch für verdeckt liegende Konkremente und eine schnelle und zuverlässige Positionierung des Patienten oder des Stosswellensystems ermöglicht.

Diese Aufgabe wird erfindungsgemäss von einer Vorrichtung mit den in den Ansprüchen genannten Merkmalen gelöst.

Erfindungsgemäss kann ein kommerzieller oder auch ein speziell angefertigter Ultraschallscanner (Ultraschallschwinger) verwendet werden. Er wird so eingesetzt, dass er in einem bestimmten Bereich, zum Beispiel dem Oberbauch, an jeder Stelle plaziert werden kann und dabei weitgehend in seiner Orientierung variabel ist, ohne dass die relative Position des Stosswellenbrennpunkts verlorengeht. Der Schwinger ist dabei annähernd so frei beweglich wie ein frei von Hand gehaltener Schwinger.

Das Aufsuchen und Darstellen von Konkrementen ist erfindungsgemäss ähnlich einfach wie in der täglichen medizinischen Praxis der Ultraschall-Diagnostik. Die Informationen über die exakte räumliche Lage des Konkrements in bezug auf einen festen Raumpunkt (Stosswellenfokus) lässt sich aus dem Ultraschallbild entnehmen, wenn gleichzeitig die drei Raumkoordinaten der aktuellen Schwingerposition so wie dessen Richtungskoordinaten registriert und verrechnet werden.

Marktübliche Compound-Scanarme zur Ultraschall-Bilderzeugung sind in der Lage, die genannten Koordinaten zu erfassen. Wird an einen derartigen Scanarm zum Beispiel ein Sektorschallkopf befestigt, so wird, anders als in der üblichen Compound-Technik, auch ohne Bewegung des Scanarmes kontinuierlich ein Ultraschallbild zur Beobachtung erzeugt.

Die Koordinaten zur Lagebestimmung werden dabei über Winkel- und Wegaufnehmer des Scanarmes bestimmt. Zusätzlich zur üblichen Technik muss eine Verrechnung dieser Koordinaten mit denen des Ziels im Ultraschallbild erfolgen. Als Ergebnis der Rechnung liegen der Abstand des Ziels vom Stosswellenfokus sowie die Richtung vor, in der das Ziel auf den Stosswellenfokus zu bewegt werden muss bzw. umgekehrt, in der der Stosswellenfokus auf das Ziel hin bewegt werden muss. Eine Verschiebung um die so ermittelten Werte bringt Zielkonkrement und Stosswellenfokus zur Deckung und ermöglicht so die gezielte Applikation von Stosswellen.

In einer vorteilhaften Ausführung wird die erfindungsgemässe Korrelation ohne eine elektronische Erfassung und Verarbeitung der notwendigen Positionierdaten mechanisch durchgeführt. Benutzt wird die Idee, dass für die Positionierung allein die Kenntnis der drei Raumkoordinaten des Konkrements X, Y und Z von Bedeutung sind. Zur Darstellung des Konkrements ist jedoch der Schwinger an verschiedenen Stellen der Körperoberfläche in möglichst freier Orientierung anzusetzen (Eintrittsfenster und Richtungen des Eintritts). Die Schwenkbarkeit des Schwingers wird vorteilhaft dadurch erreicht, dass der Schwinger sich durch eine geeignete Führung auf Kugelflächen bewegen kann, wobei der Schwinger immer auf ein festes Zentrum ausgerichtet bleibt. Eine mögliche Ausführungsform ist eine kardanische Aufhängung, wobei der Schwinger auf einem Bogen verschoben wird, und dieser Bogen selbst verschwenkbar ist. Auf diese Weise lässt sich das Zentrum (Konkrement) unter beliebigen Richtungen anpeilen; es können also die durch die Anatomie vorgegebenen Eintrittsfenster voll ausgenutzt werden.

Die Schwenkeinrichtung für den Ultraschallkopf muss ihrerseits in den drei Raumkoordinaten X, Y und Z verschiebbar sein, damit unter Anpassung an die Fenster bei freier Wahl der Beobachtungsrichtung das interessierende Konkrement mit dem Zentrum der Schwenkeinrichtung zur Deckung gebracht werden kann. Sobald das Konkrement an einer definierten Stelle im Ultraschallbild erscheint, geben allein die Koordinaten X, Y und Z die Lage des Konkrements im Raum an. An dem X-, Y-, Z-Positioniersystem kann, unabhängig von der gewählten Beobachtungsrichtung, zum Beispiel durch einen fest verbundenen Markierungsarm, die Raumposition des Konkrements relativ zur Liege (zum Patienten) markiert werden. Diese Markierung muss im nächsten Schritt lediglich mit einer zweiten Markierung, die fest mit dem Lithotripter verbunden ist, zur Deckung gebracht werden. Dies kann sehr einfach von Hand nach Sicht oder auch automatisch erfolgen.

Ohne Einschränkung der Bewegungsfreiheit sind also die Schwenkwinkel und die Raumkoordinaten X, Y und Z so miteinander gekoppelt, dass jede Bewegung des Schallkopfes aufgeteilt wird in eine Bewegung in den drei Raumkoordinaten X, Y und Z und in eine Drehung, die für die Positionierung ohne Bedeutung ist. Wesentlich ist, dass das eigentliche Zentrum der Drehung im Patientenkörper liegt und damit selbst nicht zugänglich ist.

Die erfindungsgemässe Vorrichtung eignet sich besonders für die Verwendung im Wasserbad, da dort kein unmittelbarer Körperkontakt des Schallkopfes notwendig ist und die Wasserstrecke als Vorlauf- und Ankoppelstrecke geeignet ist.

Für die Verwendung der erfindungsgemässen Vorrichtung ausserhalb des Wasserbades (in Verbindung mit einem Koppelkissen) ist es darüber hinaus notwendig, den Schallkopf in unmittelbaren Kontakt mit dem Körper zu bringen.

Dies wird dadurch erreicht, dass der Schwinger in einer axial verschieblichen Halterung erfasst wird, so dass er in Richtung auf das Zentrum der Schwenkeinrichtung so lange verschoben werden kann, bis er in Kontakt mit der Körperoberfläche gelangt. Im Ultraschallbild wandert synchron mit der axialen Schwingerverschiebung das Zentrum der Schwenkeinrichtung auf der Mittellinie des Bildes. Das Zentrum kann also nicht durch eine feste Markierung im Ultraschallbild gekennzeichnet werden, vielmehr muss die Markierung ebenfalls synchron wandern. Die aktuelle axiale Position des Schallkopfes kann über einen Wegaufnehmer festgestellt und zur Positionierung der Markierung verwendet werden.

Der Ortungsvorgang besteht nun darin, das interessierende Konkrement durch spielerische Variation von Einkoppelfenster und Orientierung des Schwingers mit der Markierung für das Zentrum der Schwenkeinrichtung zur Deckung zu bringen und in einem zweiten Schritt mit den nunmehr bekannten Raumkoordinaten das Zentrum der Schwenkeinrichtung in den Stosswellenfokus zu justieren.

Ein weiterer Vorteil der erfindungsgemässen Vorrichtung ergibt sich daraus, dass ein weiterer Freiheitsgrad wählbar bleibt. Es lässt sich nämlich die Ultraschallschnittebene durch axiale Drehung des Schallkopfes frei wählen, ohne dass dadurch der Positioniervorgang beeinflusst wird. Die Wahl der Ebene kann nach verschiedenen Gesichtspunkten erfolgen, zum Beispiel so, dass die atembedingte Bewegung der Organe wesentlich in der US-Schnittebene erfolgt und damit kontinuierlich beobachtet werden kann (getriggerte Stosswellenauslösung).

Eine andere Realisierungsmöglichkeit besteht in der Verwendung eines Roboterarmes mit einer ausreichenden Anzahl von Gelenken, so dass die drei Freiheitsgrade der Translation vorhanden sind und der Ultraschallschwinger zusätzlich in allen Raumrichtungen schwenkbar ist. Der Arm mit dem Ultraschallschwinger an der Spitze kann automatisch oder von Hand geführt werden. Sobald das Zielkonkrement im Ultraschallbild erscheint, ist seine exakte Position erfasst. Sie lässt sich durch Auswertung der Gelenkeinstellungen (Winkel) oder über Wegaufnehmer eindeutig ermitteln. Neben der jeweiligen Einstellung des Roboterarmes ist die Position des Zielobjekts im Ultraschallbild selbst zu berücksichtigen. Dies geschieht, wie bereits in herkömmlichen Geräten praktiziert, zum Beispiel durch Verschieben einer beweglichen Markierung auf dem Monitorbild an den Ort des Zielobjekts (interaktives Bildschirmsystem).

Eine Ausführungsform der Erfindung wird anhand zweier Figuren näher erläutert.

Figur 1 zeigt eine erfindungsgemässe Vorrichtung mit einem Patienten in Seitenansicht,

Figur 2 zeigt eine erfindungsgemässe Vorrichtung mit einem Patienten in Blickrichtung der Körperlängsachse.

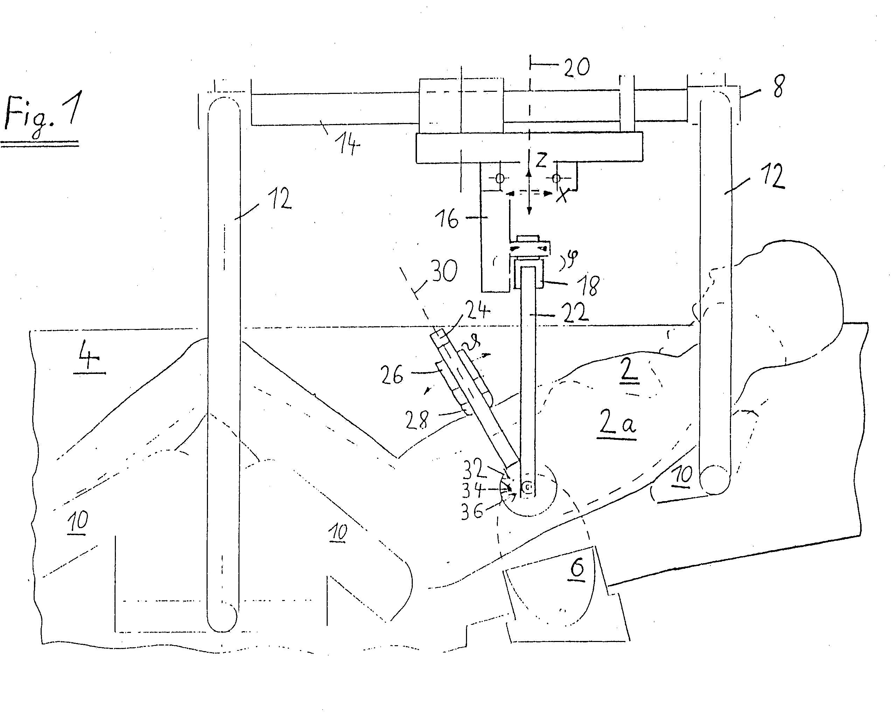

Figur 1 zeigt den Körper 2 eines Patienten in Seitenansicht, der sich in einer wassergefüllten Wanne 4 befindet. Ein kleinerer Körper 2a ist gestrichelt gezeichnet. Am Boden der Wanne 4 ist ein ellipsoidförmiger Stosswellenfokussierungskörper 6 angebracht, in dem durch Funkenentladung Stosswellen erzeugbar auf den zweiten Brennpunkt 36 fokussierbar sind. Der Körper 2 ruht auf einer Liege 8 auf Auflagekissen 10, die über Tragarme 12 an einem Querträger 14 befestigt sind. Am Querträger 14 ist auch die erfindungsgemässe Ortungs- und Positioniervorrichtung befestigt. Sie enthält in dieser Ausführung einen Träger 16, der in den drei Raumrichtungen X, Y, Z (Y senkrecht zur Bildebene) verschiebbar ist, eine daran befestigte Führung 18, die um eine vertikale Achse 20 um den Winkel φ verdreht werden kann. In der Führung 18 ist eine äussere halbkreisförmige Schiene 22 gelagert. An den Enden der Schiene 22 ist drehbar eine innere halbkreisförmige Schiene 24 gelagert, die um den Winkel ϑ verdrehbar ist. An der inneren Schiene 24 befindet sich auf einem Schlitten 27 der Ultraschallschwinger 26, der durch Federkraft über ein Ankoppelkissen 28 am Körper 2 anliegt. Der Ultraschallschwinger 26 ist zusätzlich um seine Hauptachse 30 verdrehbar und längs dieser Achse 30 verschiebbar angeordnet. Die Vorrichtung ist so konstruiert, dass die genannten Verdrehungen den Ultraschallschwinger 26 stets auf einen festen Punkt, den Mittelpunkt 32 der bogenförmigen Schienen 24 und 22 ausgerichtet lassen.

Die Ortung kann folgendermassen durchgeführt werden:

Der Arzt schaltet den Ultraschallschwinger 26 ein und betrachtet auf einem nicht gezeigten Ultraschallbildschirm ein Schnittbild des Patienten. Der Arzt kann den Ultraschallschwinger 26 spielerisch an verschiedene Körperstellen schieben (z.B. durch Translation in den Richtungen X, Y, Z oder Verkippungen um die Winkel γ, ϑ oder φ) um das Konkrement zu finden. Er kann gleichzeitig den Ultraschallschwinger 26 an jeder Körperstelle um einen gewissen Winkelbetrag verkippen, zum Beispiel um den Winkel ϑ oder durch Verschieben der äusseren Schiene 22 in der Führung 18 oder durch Verschieben des Ultraschallschwingers 26 auf der inneren Schiene 24. Der Arzt kann so ein Eintrittsfenster und eine Eintrittsrichtung für die Ultraschallstrahlung suchen (Schlüssellocheffekt). Der Abstand r des Konkrements 34 vom Ultraschallschwinger 26 ist ebenfalls einstellbar und im Ultraschallbild anzeigbar. Ein Fadenkreuz wird in dem Ultraschallbildschirm stets so mitgeführt, dass es stets dem Mittelpunkt 32 entspricht. Zusätzlich kann der Ultraschallschwinger 26 um seine eigene Hauptachse 30 gedreht werden, das heisst, wenn der Stein gefunden ist, kann der Arzt die Schnittebene nochmals verdrehen, um die Schnittebene (= Bildebene) in eine mögliche Bewegungsebene des Steins zu bringen, zum Beispiel um den Stein bei der Mitbewegung durch die Atmung zu beobachten. Der Stein ist dann während der gesamten Applikation auf dem Ultraschallbild sichtbar. Der Stein befindet sich, wenn er im Fadenkreuz des Ultraschallbildschirms liegt, im Mittelpunkt 32 der zwei kreisbogenförmigen Schienen 22 und 24. Alle möglichen Verdrehungen belassen durch die erfindungsgemässe Aufhängung den Stein im Ultraschallbildschirm am richtigen Ort.

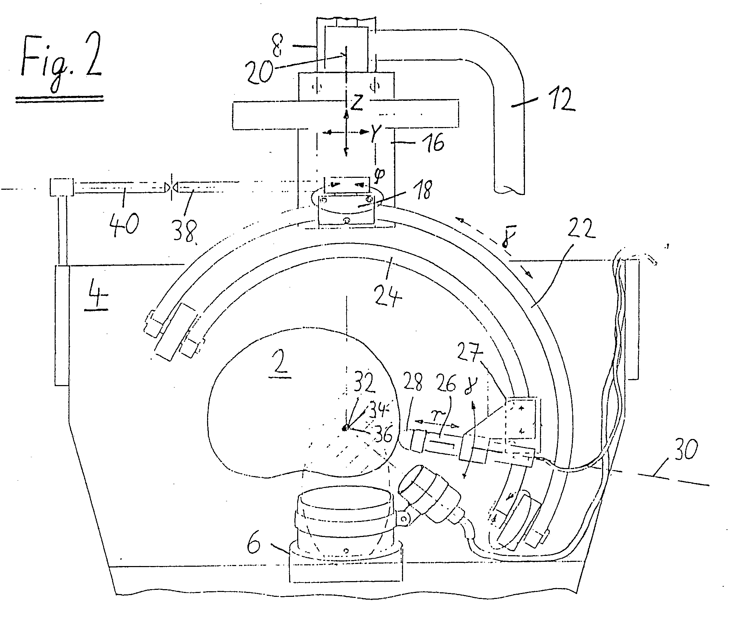

Die Positionierung geschieht dadurch, dass der Patientenkörper 2 mit der Liege 8 so gegen die Wanne 4 verschoben wird, dass der liegenfeste Mittelpunkt 32, der der Ort des Konkrements 34 ist, in den wannenfesten zweiten Brennpunkt 36 der Stosswellenfokussierungskammer 6 gebracht wird. Dies geschieht durch Verschieben der Liege 4 bis zwei in Figur 2 gezeigte Markierungen in drei Dimensionen aneinander liegen.

Das Konkrement 34 (hier ein Nierenstein) ist nun an der richtigen Stelle, um durch Stosswellen aus der Stosswellenfokussierungskammer 6 zerkleinert zu werden. Der Erfolg der Zerkleinerung des Konkrements 34 kann über Ultraschall beobachtet werden.

Figur 2 zeigt die Vorrichtung der Figur 1 in Blickrichtung der Körperlängsachse des Patienten 2. Deutlich zu erkennen sind die bogenförmigen Schienen 22 und 24, wobei die Schiene 22 in der Führung 18 läuft (Drehmöglichkeit um den Winkel γ̄) und auf der Schiene 24 der Ultraschallschwinger 26 mit dem Schlitten 27 beweglich befestigt ist (Drehmöglichkeit γ). Zu erkennen ist ebenfalls die Bewegungsmöglichkeit des Ultraschallschwingers 26 längs seiner Achse 30 (Pfeil r). An Träger 16 ist ein Markierungsstift 38 angebracht, dessen Spitze vom Mittelpunkt 32, dem Steinort bei richtiger Ortung, eine in Richtung und Betrag definierte Entfernung hat. Er ist fest gegen den Mittelpunkt 32, aber gegen die Wanne 4 beweglich angebracht.

Der zweite Markierungsstift 40 ist fest an der Wanne 4 angebracht. Seine Spitze hat vom zweiten Brennpunkt 36 der Fokussierungskammer 6 die in Betrag und Richtung gleiche Entfernung wie die Spitze des ersten Markierungsstifts 38 vom Punkt 32. Das heisst, die Positionierung erfolgt nach der Ortung durch einfaches Verschieben der Liege 8 so, dass die beiden Spitzen der Markierungsstifte 38, 40 aneinanderliegen.

An der Stosswellenfokussierungskammer 6 ist rechts ein zweiter Ultraschallkopf eingezeichnet. Dieser zweite Ultraschallkopf ist fest auf den zweiten Brennpunkt 36 ausgerichtet, kann dabei aber um die Hochachse der Stosswellenfokussierungskammer 6 drehbar sein (um ein Eintrittsfenster auswählen zu können), hat eine höhere Auflösung als der bewegliche Ultraschallschwinger 26 und erlaubt eine zusätzliche, genauere Endjustierung und Überwachung der Zerkleinerung des Konkrements 34. Die höhere Auflösung kann durch eine grössere Apertur oder durch den Verzicht auf Tiefenschärfe erreicht werden.

## Patentansprüche

1. Ortungs- und Positioniervorrichtung für die berührungsfreie Zerkleinerung von Konkrementen in Körpern von Lebewesen mit einem Ortungssystem, das über einen grösseren Bereich des Körpers (2) verschiebbar ist, mit Vorrichtungen (38, 40), die die Position oder die Position und die Richtung des Ortungssystems markieren oder registrieren und eine mechanische Korrelation der Position des Konkrements (34) mit dem Brennpunkt (36) eines Stosswellensystems ermöglichen, dadurch gekennzeichnet, dass das Ortungssystem ein Ultraschallschwinger (26) ist, der an einer Führung verschiebbar ist, dass die Führung den Ultraschallschwinger (26) bei allen Bewegungen stets auf einen festen Mittelpunkt (32) ausgerichtet hält und dass zwei Markierungsstifte (38, 40) vorgesehen sind, von denen der eine (38) eine feste Position gegenüber dem Mittelpunkt (32) innehat und der andere (40) eine feste Position gegenüber dem Stosswellenfokus (36) innehat.

2. Ortungs- und Positioniervorrichtung für die berührungsfreie Zerkleinerung von Konkrementen in Körpern von Lebewesen mit einem Ortungssystem, dass über einen grösseren Bereich des Körpers (2) verschiebbar ist, und mit Vorrichtungen, die die Position oder die Position und die Richtung des Ortungssystems markieren oder registrieren und eine elektronische Korrelation der Position des Konkrements (34) mit dem Brennpunkt (36) eines Stosswellensystems ermöglichen, dadurch gekennzeichnet, dass das Ortungssystem ein Ultraschallschwinger (26) ist, der an einer Führung verschiebbar ist, dass die Führung den Ultraschallschwinger (26) bei allen Bewegungen stets auf einen festen Mittelpunkt (32) ausgerichtet hält und dass zur elektronischen Korrelation Winkelgeber und/oder Wegaufnehmer vorgesehen sind, die die Positionsdaten und die Einstellwinkel des Ultraschallschwingers (26) registrieren.

3. Vorrichtung nach Anspruch 1 oder 2, dadurch gekennzeichnet, dass der Ultraschallschwinger (26) auf einem Schlitten (27) auf einer ersten inneren kreisbogenförmigen Schiene (24) beweglich ist, dass die innere kreisbogenförmige Schiene (24) an den Enden einer zweiten äusseren kreisbogenförmigen Schiene (22) drehbar gelagert ist, dass die äussere kreisbogenförmige Schiene (22) in einer Führung verschiebbar ist, dass die Führung (18) um eine vertikale Achse (20) verdrehbar ist und dass die Führung (18) gegen einen Träger (16) in drei Raumrichtungen (X, Y, Z) verschiebbar ist.

4. Vorrichtung nach Anspruch 3, dadurch gekennzeichnet, dass die Hauptachse des Ultraschallschwingers (26), die Verschwenkachse für die innere Schiene (24) und die Drehachse der Führung (18) sich im Mittelpunkt (32) der zwei Kreisbögen (22, 24) schneiden.

5. Vorrichtung nach einem der vorhergehenden Ansprüche, dadurch gekennzeichnet, dass der Ultraschallschwinger (26) auf einem Schlitten (27) längsbeweglich in Richtung der Ultraschallschwingerhauptachse (30) befestigt ist und dass ein Positionsgeber vorhanden ist, der eine Anzeige des Mittelpunkts (32) im Ultraschallbild selbsttätig der jeweiligen Position des Ultraschallschwingers (26) nachführt.

6. Vorrichtung nach einem der vorhergehenden Ansprüche, dadurch gekennzeichnet, dass die Positionierung durch zur Deckung bringen von Stosswellenfokus (36) und Mittelpunkt (32) automatisch erfolgt.

7. Vorrichtung nach Anspruch 6, dadurch gekennzeichnet, dass die Positionierung durch Verschieben einer Markierung auf dem Monitorbild an den Ort des Konkrements (34) automatisch erfolgt.

8. Vorrichtung nach einem der vorhergehenden Ansprüche, dadurch gekennzeichnet, dass ein zweiter, auf den zweiten Brennpunkt (36) der Stosswellenfokussierungskammer (6) gerichteter Ultraschallkopf mit hoher Auflösung vorgesehen ist.

## Claims

1. Locating and positioning device for the contact free breaking down of concrements in bodies of living organisms, comprising a locating system which is displaceable over a relatively wide region of the body (2), devices (38, 40) which mark or register the position or the position and direction of the locating system and enable the position of the concrement (34) to be mechanically correlated with the focus (36) of a system of shock waves, characterised in that the locating system is an ultrasound vibrator (26) which is displaceable on a guide, and that the guide keeps the ultrasound vibrator (26) orientated to a fixed center point (32) in all movements and in that two marking pencils (38, 40) are provided, one (38) of which takes up a fixed position in relation to the center point (32) while the other (40) takes up a fixed position in relation to the shock wave focus (36).

2. Locating and positioning device for the contact free breaking down of concrements in bodies of living organisms, comprising a locating system which is displaceable over a relatively wide range of the body (2) and devices which mark or register the position or the position and direction of the locating system and enable the position of the concrement (34) to be electronically correlated with the focus (36) of a system of shock waves, characterised in that the locating system is an ultrasound vibrator (26) which is displaceable on a guide, in that the guide keeps the ultrasound vibrator (26) orientated to a fixed center point (32) in all movements and in that angle transmitters and/or path transducers which record the position data and setting angle of the ultrasound vibrator (26) are provided for the electronic correlation.

3. Device according to Claim 1 or 2, characterised in that the ultrasound vibrator (26) is displaceable on a first inner rail (24) in the form of a circular arc on a carriage (27) in that the inner rail (24) in the form of a circular arc is rotatably mounted at the ends of a second, outer rail (22) in the form of a circular arc, in that the outer rail (22) in the form of a circular arc is displaceable in a guide, in that the guide (18) is rotatable about a vertical axis (20) and in that the guide (18) is displaceable in three directions in space (X, Y, Z) in relation to a support (16).

4. Device according to Claim 3, characterised in that the major axis of the ultrasound vibrator (26) intersects the axis of swing of the inner rail (24) and the axis of rotation of the guide (18) at the center point (32) of the two cirular arcs (22, 24).

5. Device according to one of the preceding Claims, characterised in that the ultrasound vibrator (26) is fixed to a carriage (27) to be longitudinally displaceable in the direction of the major axis (30) of the ultrasound vibrator and in that a position transmitter is present which automatically moves the indication of the center point (32) in the ultrasound image to follow the position of the ultrasound vibrator (26).

6. Device according to one of the preceding Claims, characterised in that positioning takes place automatically by the shock wave focus (36) and the center point (32) being made to coincide.

7. Device according to Claim 6, characterised in that the positioning takes place automatically by displacement of a mark on the screen image to the position of the concrement (34).

8. Device according to one of the preceding Claims, characterised in that a second ultrasound head with high resolution directed to the second focus (36) of the sound wave focusing chamber (6) is provided.

## Revendications

1. Dispositif de détection et de positionnement pour la fragmentation sans contact de concrétions à l'intérieur des corps d'êtres vivants, avec un système de détection pouvant être déplacé sur une grande partie du corps (2), et avec des dispositifs (38, 40) qui repèrent ou enregistrent la position ou la position et l'orientation du système de détection et permettent d'établir une corrélation mécanique entre la position de la concrétion (34) et le foyer (36) d'un système à ondes de choc, caractérisé en ce que le système de détection est un oscillateur ultrasonore (26) pouvant être déplacé sur un guidage, que le guidage assure toujours, pour tous les mouvements, le pointage de l'oscillateur ultrasonore (26) vers un centre fixe (32), et qu'il est prévu deux pointes de repérage (38, 40) dont l'une (38) occupe une position fixe par rapport au centre (32), tandis que l'autre (40) occupe une position fixe par rapport au foyer des ondes de choc (36).

2. Dispositif de détection et de positionnement pour la fragmentation sans contact de concrétions à l'intérieur des corps d'êtres vivants, avec un système de détection pouvant être déplacé sur une grande partie du corps (2), et avec des dispositifs qui repèrent ou enregistrent la position ou la position et l'orientation du système de détection et permettent d'établir une corrélation électronique entre la position de la concrétion (34) et le foyer (36) d'un système à ondes de choc, caractérisé en ce que le dispositif de détection est un oscillateur ultrasonore (26) pouvant être déplacé sur un guidage, que le guidage assure toujours, pour tous les mouvements, le pointage de l'oscillateur ultrasonore (26) vers un centre fixe (32), et que pour la corrélation électronique, il est prévu des capteurs angulaires et/ou des capteurs de déplacement qui enregistrent les données de position et les angles de réglage de l'oscillateur ultrasonore (26).

3. Dispositif selon l'une des revendications 1 ou 2, caractérisé en ce que l'oscillateur ultrasonore (26) peut être déplacé sur un chariot (27), sur un premier rail intérieur (24) en forme d'arc de cercle, que le rail intérieur (24) en arc de cercle est monté de manière tournante aux extrémités d'un second rail extérieur (22) en forme d'arc de cercle, que le rail extérieur (22) en arc de cercle peut être déplacé dans un guidage, que le guidage (18) peut tourner autour d'un axe vertical (20), et que le guidage (18) peut être déplacé dans trois directions spatiales (X, Y, Z) par rapport à un support (16).

4. Dispositif selon la revendication 3, caractérisé en ce que l'axe principal de l'oscillateur ultrasonore (26), l'axe de pivotement pour le rail intérieur (24) et l'axe de rotation du guidage (18) se coupent au centre (32) des deux arcs de cercle (22, 24).

5. Dispositif selon l'une des revendications précédentes, caractérisé en ce que l'oscillateur ultrasonore (26) est fixé sur un chariot (27) de façon à pouvoir être déplacé dans le sens longitudinal dans la direction de l'axe principal (30) de l'oscillateur ultrasonore, et qu'il est prévu un capteur de position qui suit automatiquement l'affichage du centre (32) dans l'image ultrasonore en fonction de la position respective de l'oscillateur ultrasonore (26).

6. Dispositif selon l'une des revendications 1 à 5, caractérisé en ce que le positionnement est effectué automatiquement par la mise en coïncidence du foyer des ondes de choc (36) et du centre (32).

7. Dispositif selon la revendication 6, caractérisé en ce que le positionnement est effectué automatiquement en déplaçant un repère sur le moniteur jusqu'à l'endroit de la concrétion (34).

8. Dispositif selon l'une des revendications précédentes, caractérisé en ce qu'il comprend une seconde tête ultrasonore de haute résolution orientée vers le second foyer (36) de la chambre de focalisation des ondes de choc (6).

Fig. 1

# Fig. 2